# EUROPÄISCHE PATENTANMELDUNG

(11) **EP 4 302 812 A1**
(43) Veröffentlichungstag der Anmeldung: **10.01.2024**
(21) Anmeldenummer: 23173728.9
(22) Anmeldetag: 16.05.2023
(51) Int. Cl.: A61M 25/00, A61B 17/34, A61M 25/02, A61M 39/06

(54) **KATHETERANORDNUNG ZUR VERWENDUNG BEI EINER PERIPHEREN REGIONALANÄSTHESIE**

(30) Priorität: 23.05.2022 DE 102022205130
(71) Anmelder: B. Braun Melsungen AG, 34212 Melsungen (DE)
(72) Erfinder: Blum, Iris, 34277 Fuldabrück (DE); Weiß, André, 34302 Guxhagen (DE); Bode, Nicky, 37308 Schimberg (DE); Felmeden, Jacqueline, 34242 Melsungen (DE)
(74) Vertreter: Patentanwälte Ruff, Wilhelm, Beier, Dauster & Partner mbB

(57) **Zusammenfassung**

Katheteranordnung (1), aufweisend ein Kapillar (100) mit einem Kapillarrohr (101) und einem Kapillaransatz (102), welcher an ein proximales Rohrende (103) des Kapillarrohrs angefügt ist, einen Katheterschlauch (200), welcher in distaler Richtung durch den Kapillaransatz in das Kapillarrohr eingeschoben ist, wobei ein distales Schlauchende (201) des Katheterschlauchs um eine axiale Länge aus einem distalen Rohrende des Kapillarrohrs hervorsteht, wobei eine Fixiervorrichtung (300) mit einem Betätigungselement (301) und einem Klemmelement (302) vorhanden ist, wobei das Klemmelement an dem Kapillaransatz gelagert ist, wobei das Betätigungselement relativ zu dem Kapillaransatz beweglich an dem Kapillaransatz gelagert und kraft- und/oder bewegungsübertragend mit dem Klemmelement wirkverbunden, und wobei das Klemmelement mittels einer Bewegung des Betätigungselements überführbar ist zwischen einer Klemmkonfiguration, in welcher eine axiale Beweglichkeit des Katheterschlauchs unter Einwirkung des Klemmelements fixiert ist, und einer Freigabekonfiguration, in welcher die Einwirkung aufgehoben und die axiale Beweglichkeit des Katheterschlauchs freigegeben ist

## Beschreibung

Die Erfindung betrifft eine Katheteranordnung zur Verwendung bei einer peripheren Regionalanästhesie, aufweisend ein Kapillar mit einem Kapillarrohr und einem Kapillaransatz, welcher an ein proximales Rohrende des Kapillarrohrs angefügt ist, einen Katheterschlauch, welcher in distaler Richtung durch den Kapillaransatz in das Kapillarrohr eingeschoben ist, wobei ein distales Schlauchende des Katheterschlauchs um eine axiale Länge aus einem distalen Rohrende des Kapillarrohrs hervorsteht.

Derartige Katheteranordnungen sind im Bereich der Regionalanästhesie allgemein bekannt und werden zur gezielten, reversiblen Blockade einzelner Nerven verwendet. In diesem Zusammenhang wird auch von einer Nervenblockade oder einem Nervenblock gesprochen. Die Nervenblockade wird durch die Verabreichung eines Anästhetikums in unmittelbarer Nähe des betreffenden Nervs hervorgerufen. Die bekannte Katheteranordnung weist hierfür ein Kapillar mit einem Kapillarrohr und einem Kapillaransatz sowie einen Katheterschlauch auf. Das Kapillar kann auch als Kurzkatheter bezeichnet werden und wird üblicherweise gemeinsam mit einer durch das Kapillarrohr distal vorgeschobenen Kanüle am Patienten appliziert. Nach Zurückziehen und Entfernen der Kanüle verbleibt das Kapillarrohr am Patienten und dient der Aufnahme und Führung des Katheterschlauchs. Um den zu blockierenden Nerv zu erreichen, wird das distale Schlauchende durch den Kapillaransatz in distaler Richtung in das Kapillarrohr eingeschoben. Bei der bekannten Katheteranordnung ist die axiale Länge, d.h. der axiale Überstand des distalen Schlauchendes über das distale Rohrende, konstruktiv festgelegt. Der Überstand ist folglich stets gleich und unveränderlich.

Aufgabe der Erfindung ist es, eine Katheteranordnung der eingangs genannten Art bereitzustellen, welche eine verbesserte Regionalanästhesie ermöglicht.

Diese Aufgabe wird dadurch gelöst, dass eine Fixiervorrichtung mit einem Betätigungselement und einem Klemmelement vorhanden ist, wobei das Klemmelement an dem Kapillaransatz gelagert ist, wobei das Betätigungselement relativ zu dem Kapillaransatz beweglich an dem Kapillaransatz gelagert und kraft- und/oder bewegungsübertragend mit dem Klemmelement wirkverbunden ist, und wobei das Klemmelement mittels einer Bewegung des Betätigungselements überführbar ist zwischen einer Klemmkonfiguration, in welcher eine axiale Beweglichkeit des Katheterschlauchs unter Einwirkung des Klemmelements fixiert ist, und einer Freigabekonfiguration, in welcher die Einwirkung aufgehoben ist und die axiale Beweglichkeit des Katheterschlauchs freigegeben ist. Durch die erfindungsgemäße Lösung kann der Überstand des distalen Schlauchendes situationsgerecht angepasst und nach erfolgter Anpassung fixiert werden. Dies ermöglicht eine besonders präzise Positionierung des distalen Schlauchendes. Das aus dem distalen Schlauchende abzugebende Anästhetikum kann hierdurch örtlich besonders präzise an den zu blockierenden Nerv abgegeben werden. Hierdurch wird eine verbesserte Schmerztherapie ermöglicht. Die erfindungsgemäß vorhandene Fixiervorrichtung ermöglicht die besagte Längenänderung und Fixierung des Katheterschlauchs und weist zu diesem Zweck das besagte Betätigungselement und das Klemmelement auf. Das Klemmelement ist zwischen der Klemm- und der Freigabekonfiguration überführbar, beispielsweise mittels einer translatorischen und/oder rotatorischen Bewegung und/oder Deformation. Die Deformation kann elastisch, plastisch und/oder elastoplastisch sein. In der Klemmkonfiguration ist der Katheterschlauch unter Einwirkung des Klemmelements fixiert, genauer reib- und/oder formschlüssig gehalten. In der Freigabekonfiguration ist die Einwirkung des Klemmelements auf den Katheterschlauch aufgehoben, so dass der Katheterschlauch zur möglichst präzisen Positionierung des distalen Schlauchendes weiter distal vorgeschoben und/oder proximal zurückgezogen werden kann. Das Klemmelement ist wenigstens mittelbar an dem Kapillaransatz gelagert. Bei einer Ausgestaltung ist das Klemmelement an einem proximalen Ende des Kapillaransatzes gelagert. Bei einer weiteren Ausgestaltung ist das Klemmelement alternativ oder zusätzlich in einer, insbesondere proximal offenen, Aufnahmeaussparung des Kapillaransatzes aufgenommen. Das Betätigungselement ist beweglich an dem Kapillaransatz gelagert. Bei einer Ausgestaltung ist das Betätigungselement drehbeweglich, insbesondere schraubbeweglich. Bei einer weiteren Ausgestaltung ist das Betätigungselement schwenkbeweglich. Bei einer weiteren Ausgestaltung ist das Betätigungselement linearbeweglich. Die Bewegung und/oder Deformation des Klemmelements zwischen der Klemm- und der Freigabekonfiguration wird durch eine manuelle Betätigungsbewegung des Betätigungselements hervorgerufen. Das Betätigungselement ist hierfür kraft- und/oder bewegungsübertragend mit dem Klemmelement wirkverbunden. Die Wirkverbindung ist bei einer Ausgestaltung unmittelbar und/oder direkt. Bei einer weiteren Ausgestaltung ist die Wirkverbindung mittelbar und/oder indirekt. Bei einer Ausgestaltung sind das Klemmelement und das Betätigungselement separate Bauteile, wobei die Wirkverbindung kraft-, form- und/oder stoffschlüssig sein kann. Bei einer weiteren Ausgestaltung ist das Betätigungselement einstückig zusammenhängend mit dem Klemmelement ausgebildet und umgekehrt, so dass die besagten Elemente unterschiedliche Abschnitte eines einzigen Bauteils bilden.

In Ausgestaltung der Erfindung ist das Klemmelement formnachgiebig und weist ein koaxial zu dem Kapillarrohr ausgerichtetes Lumen auf, durch welches der Katheterschlauch längserstreckt ist, wobei das Klemmelement mittels der Bewegung des Betätigungselements zwischen der Klemmkonfiguration und der Freigabekonfiguration elastisch deformierbar ist. In der Klemmkonfiguration ist das Lumen infolge der elastischen Deformation radial verengt, wodurch der Katheterschlauch reibschlüssig in dem Lumen fixiert ist. In der Freigabekonfiguration ist der Reibschluss aufgehoben. Bei dieser Ausgestaltung ist das Klemmelement zwischen der Klemm- und der Freigabekonfiguration elastisch deformierbar. In der Klemmkonfiguration ist der Katheterschlauch reibschlüssig in dem Lumen des Klemmelements gehalten, genauer geklemmt. In der Freigabekonfiguration ist diese Klemmung aufgehoben, so dass der Katheterschlauch zur möglichst präzisen Positionierung des distalen Schlauchendes weiter distal vorgeschoben und/oder proximal zurückgezogen werden kann. Bei einer Ausgestaltung ist die Formnachgiebigkeit/elastische Deformierbarkeit durch eine entsprechende Materialwahl erreicht. Beispielsweise kann das Klemmelement aus einem elastomeren Kunststoffmaterial gefertigt sein. Bei einer weiteren Ausgestaltung ist die Formnachgiebigkeit/elastische Deformierbarkeit des Klemmelements gestaltungsbedingt, beispielsweise kann das Klemmelement dünnwandig gestaltet sein. Die elastische Deformation des Klemmelements wird durch eine manuelle Betätigungsbewegung des Betätigungselements hervorgerufen.

In weiterer Ausgestaltung der Erfindung ist das Betätigungselement axial schraubbeweglich an dem Kapillaransatz gelagert und weist einen Gewindeabschnitt auf, welcher mit einem komplementären Gewindeabschnitt des Kapillaransatzes zusammenwirkt. Der Gewindeabschnitt und der komplementäre Gewindeabschnitt sind folglich axial orientiert. Die schraubbewegliche Lagerung des Betätigungselements ist konstruktiv einfach umsetzbar. Zudem ergibt sich eine ergonomisch vorteilhafte Betätigung. Die zur Bewegung und/oder Deformation des Klemmelements erforderlichen Kräfte und/oder Bewegungen können mittels des schraubbeweglich gelagerten Betätigungselements einerseits leichtgängig und andererseits besonders präzise aufgebracht werden.

In weiterer Ausgestaltung der Erfindung ist der Gewindeabschnitt als Innengewinde gestaltet und der komplementäre Gewindeabschnitt ist als Außengewinde gestaltet. Durch eine solche Zuordnung von Innen- und Außengewinde zu den unterschiedlichen Komponenten der Katheteranordnung können insbesondere eine vereinfachte Fertigung und Montage erreicht werden.

In weiterer Ausgestaltung der Erfindung weist das Betätigungselement einen Kragenabschnitt auf, welcher koaxial zu dem Lumen orientiert ist und ein proximales Ende des Kapillaransatzes in Umfangsrichtung umgreift. Der Kragenabschnitt ermöglicht eine ergonomisch vorteilhafte Betätigung des Betätigungselements. Sofern das Betätigungselement ein Innengewinde gemäß der vorhergehenden Ausgestaltung aufweist, ist der Kragenabschnitt vorzugsweise koaxial zu dem Innengewinde orientiert.

In weiterer Ausgestaltung der Erfindung ist das Klemmelement in einer proximal offenen Aufnahmeaussparung des Kapillaransatzes aufgenommen. Die Aufnahmeaussparung ist vorzugsweise koaxial zu dem Kapillarrohr und/oder dem Lumen orientiert. Durch diese Ausgestaltung können axial kompakte Abmessungen des Kapillaransatzes erreicht werden. Dies im Unterschied zu einer Ausgestaltung, bei welcher das Klemmelement an einem proximalen Ende, insbesondere proximalem Stirnende, des Kapillaransatzes gelagert ist.

In weiterer Ausgestaltung der Erfindung weist das Klemmelement eine koaxial zu dem Lumen orientierte und distal sich verjüngende Außenkonusfläche auf, wobei in der Klemmkonfiguration die Außenkonusfläche gegen eine Innenkonusfläche der Aufnahmeaussparung gepresst ist. Durch das Anpressen der Außenkonusfläche an die Innenkonusfläche wird das Klemmelement in die Klemmkonfiguration elastisch deformiert. Die Anpressbewegung bewirkt die radiale Verengung des Lumens. Die Innenkonusfläche ist wenigstens abschnittsweise komplementär zu der Außenkonusfläche. In der Freigabekonfiguration ist die Außenkonusfläche nicht oder weniger stark gegen die Innenkonusfläche gepresst. Eine Betätigungsbewegung des Betätigungselements bewirkt bei dieser Ausgestaltung eine Relativbewegung zwischen der Außenkonus- und der Innenkonusfläche. Bei einer Betätigung in Richtung der Klemmkonfiguration wird die Außenkonusfläche relativ zu der Innenkonusfläche distal verlagert. Bei einer Betätigungsbewegung in Richtung der Freigabekonfiguration wird die Außenkonusfläche relativ zu der Innenkonusfläche proximal verlagert.

In weiterer Ausgestaltung der Erfindung ist das Lumen zwischen einer proximalen Einführöffnung mit einem ersten Durchmesser und einer distalen Austrittsöffnung mit einem zweiten Durchmesser längserstreckt, wobei der erste Durchmesser größer ist als der zweite Durchmesser. Der in Relation größere erste Durchmesser erlaubt insbesondere ein vereinfachtes Einführen des distalen Schlauchendes in das Lumen. Vorzugsweise ist das Lumen gerade längserstreckt. Vorzugsweise ist die distale Austrittsöffnung proximal von dem proximalen Rohrende des Kapillarrohrs beabstandet.

In weiterer Ausgestaltung der Erfindung mündet die proximale Einführöffnung in einen distal sich verjüngenden Innenkonus des Lumens. Der Innenkonus ermöglicht ein nochmals vereinfachtes Einführen des Katheterschlauchs.

In weiterer Ausgestaltung der Erfindung ist das Betätigungselement axial formschlüssig und in Umfangsrichtung gleitbeweglich an dem Klemmelement gelagert. Der Formschluss wirkt wenigstens in distaler Richtung. Vorzugsweise wirkt der Formschluss axial beidseitig, d.h. distal und proximal. Bei dieser Ausgestaltung sind das Betätigungselement und das Klemmelement separat gefertigte - in diesem Fall formschlüssig - miteinander zusammengefügte Bauteile.

In weiterer Ausgestaltung der Erfindung weist das Klemmelement einen proximal angeordneten Lagerabschnitt mit einer koaxial zu dem Lumen orientierten Gleitfläche und einer die Gleitfläche distal begrenzenden Anlaufschulter auf, wobei der Lagerabschnitt eine koaxial zu dem Lumen orientierte Bohrung des Betätigungselements axial durchgreift, und wobei eine Innenumfangsfläche der Bohrung gleitbeweglich auf der Gleitfläche abgestützt ist. Diese Ausgestaltung erlaubt konstruktive und funktionelle Vorteile. Die Gleitfläche und die Innenumfangsfläche dienen der gleitbeweglichen Lagerung des Betätigungselements an dem Klemmelement. Die Gleitfläche ist vorzugsweise eine Außenumfangsfläche. Ein Innendurchmesser der Innenumfangsfläche und ein Außendurchmesser der Gleitfläche sind vorzugsweise nach Art einer Spielpassung aufeinander abgestimmt. Der Innendurchmesser ist folglich geringfügig größer als der Außendurchmesser. Die Anlaufschulter dient dem axialen Formschluss des Betätigungselements. Bei einer Betätigungsbewegung in Richtung der Klemmkonfiguration drückt das Betätigungselement in distaler Richtung auf die Anlaufschulter. Bei einer Ausgestaltung ist die Anlaufschulter ein in Umfangsrichtung durchgängig umlaufender Radialbund. Bei einer weiteren Ausgestaltung ist die Anlaufschulter durch mehrere in Umfangsrichtung versetzt angeordnete radiale Vorsprünge gebildet.

In weiterer Ausgestaltung der Erfindung ist das Klemmelement aus einem elastomeren Kunststoffmaterial gefertigt und/oder das Betätigungselement ist aus einem im Vergleich zu dem Klemmelement formstabilen Kunststoffmaterial gefertigt. Die besagte Materialwahl unterstützt eine anforderungsgerechte Formnachgiebigkeit/elastische Deformierbarkeit des Klemmelements.

Weitere Vorteile und Merkmale der Erfindung ergeben sich aus den Ansprüchen sowie aus der nachfolgenden Beschreibung eines bevorzugten Ausführungsbeispiels der Erfindung, welches anhand der Zeichnungen dargestellt ist.
- Fig. 1: zeigt in schematischer Seitenansicht eine Ausführungsform einer erfindungsgemäßen Katheteranordnung mit einem Kapillar, einem Katheterschlauch und einer Fixiervorrichtung,
- Fig. 2: die Katheteranordnung nach Fig. 1 in einer schematischen und teilweise unterbrochenen Längsschnittdarstellung entlang einer Schnittlinie A-A gemäß Fig. 1 und
- Fig. 3: die Fixiervorrichtung der Katheteranordnung nach den Fig. 1 und 2 in einer teilweisen Längsschnittdarstellung.

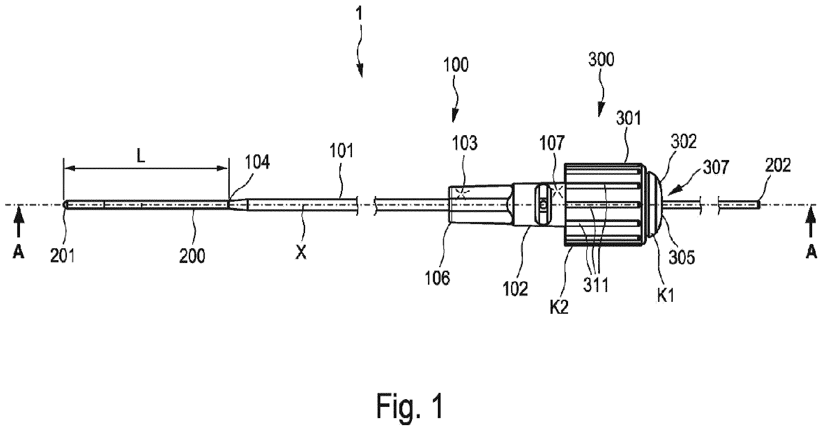

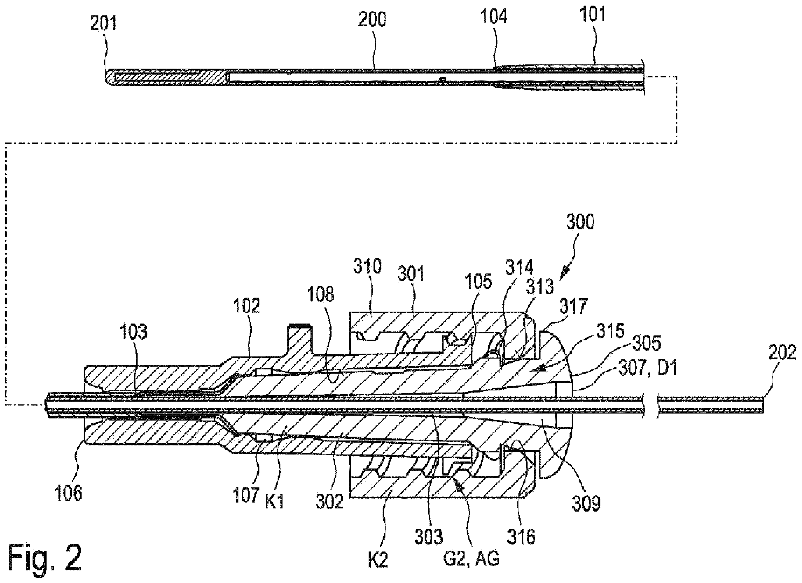

Gemäß den Fig. 1 und 2 ist eine Katheteranordnung 1 zur Verwendung bei einer peripheren Regionalanästhesie vorgesehen.

Die Katheteranordnung 1 weist ein Kapillar 100, einen Katheterschlauch 200 und eine Fixiervorrichtung 300 auf.

Das Kapillar 100 weist ein Kapillarrohr 101 und einen Kapillaransatz 102 auf.

Das Kapillarrohr 101 kann auch als Kurzkatheter bezeichnet werden. Das Kapillarrohr 101 weist ein proximales Rohrende 103 und ein distales Rohrende 104 auf. Das Kapillarrohr 101 ist zwischen dem proximalen Rohrende 103 und dem distalen Rohrende 104 gerade längserstreckt.

Der Kapillaransatz 102 ist an das proximale Rohrende 103 angefügt (siehe Fig. 2). Bei der gezeigten Ausführungsform ist eine stoffschlüssige Fügeverbindung vorgesehen. Beispielsweise kann der Katheteransatz 102 mit dem proximalen Rohrende 103 verschweißt und/oder verklebt sein.

Der Katheterschlauch 200 ist zwischen einem distalen Schlauchende 201 und einem proximalen Schlauchende 202 längserstreckt. Der Katheterschlauch 200 ist in distaler Richtung durch den Kapillaransatz 102 in das Kapillarrohr 101 eingeschoben, wobei das distale Schlauchende 201 um eine axiale Länge L aus dem distalen Rohrende 104 des Kapillarrohrs 101 hervorsteht.

Die axiale Länge L (Überstand) ist situationsgerecht anpassbar und nach erfolgter Anpassung fixierbar. Zu diesem Zweck ist die Fixiervorrichtung 300 vorhanden.

Die Fixiervorrichtung 300 weist ein Betätigungselement 301 und ein Klemmelement 302 auf.

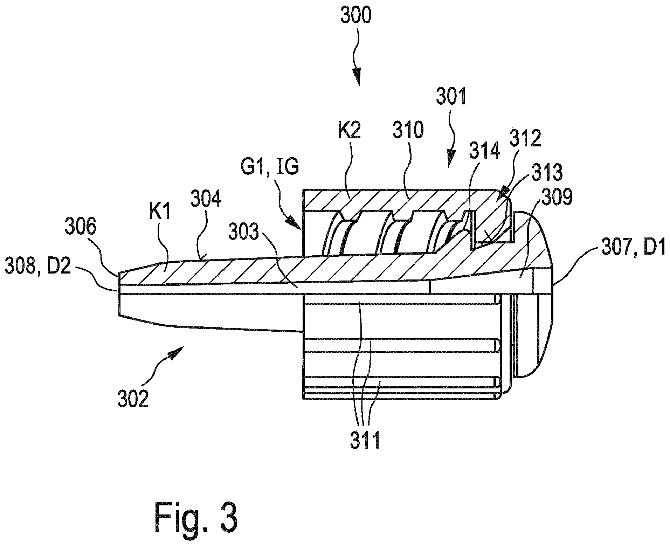

Das Klemmelement 302 ist an dem Kapillaransatz 102 gelagert. Details der Lagerung werden nachfolgend noch näher beschrieben. Weiter weist das Klemmelement 302 vorliegend ein Lumen 303 auf. Das Lumen 303 (siehe Fig. 3) ist koaxial zu dem Kapillarrohr 101 und folglich auch koaxial zu dem Katheterschlauch 202 längserstreckt. Der Katheterschlauch 200 ist in dem Lumen 303 aufgenommen und/oder durch dasselbe längserstreckt (siehe Fig. 2).

Das Betätigungselement 301 ist relativ zu dem Kapillaransatz 102 beweglich an dem Kapillaransatz 102 gelagert. Zudem ist das Betätigungselement 301 kraft- und/oder bewegungsübertragend mit dem Klemmelement 302 verbunden. Die im Hinblick auf die gezeigte Ausführungsform spezifische Umsetzung der beweglichen Lagerung einerseits und der Wirkverbindung andererseits werden nachfolgend noch näher beschrieben.

Das Klemmelement 302 ist bei der gezeigten Ausführungsform formnachgiebig. Das Klemmelement 302 ist mittels einer Bewegung des Betätigungselements 301 zwischen einer Klemmkonfiguration und einer Freigabekonfiguration elastisch deformierbar. In der Klemmkonfiguration ist das Klemmelement 302 derart elastisch deformiert, dass das Lumen 303 radial verengt ist und die Beweglichkeit des Katheterschlauchs 202 reibschlüssig in dem Lumen 303 fixiert ist. In den Fig. 1 und 2 ist die besagte Klemmkonfiguration gezeigt. In der Freigabekonfiguration ist der Reibschluss zwischen dem Lumen 303 und Katheterschlauch 200 aufgehoben und die axiale Beweglichkeit des Katheterschlauchs 200 relativ zu dem Lumen 303 und damit auch dem Kapillar 100 ist freigegeben.

Bei einer in den Figuren nicht gezeigten Ausführungsform wirkt das Klemmelement in der Klemmkonfiguration nach Art eines Exzenters auf den Katheterschlauch. Bei dieser nicht gezeigten Ausführungsform ist das Klemmelement nicht formnachgiebig und weist kein Lumen auf.

Das Kapillarrohr 101, der Kapillaransatz 102, der Katheterschlauch 200 und das Lumen 303 sind vorliegend koaxial zu einer gemeinsamen Längsachse X ausgerichtet.

Nachfolgend werden weitere gegenständliche und funktionelle Merkmale der gezeigten Ausführungsform erläutert. Die erläuterten Merkmale sind vorteilhaft, aber im Hinblick auf die vorliegende Erfindung nicht unbedingt wesentlich.

Bei der gezeigten Ausführungsform ist der Kapillaransatz 102 zwischen einem proximalen Ende 105 und einem distalen Ende 106 längserstreckt. Das Klemmelement 302 und/oder das Betätigungselement 301 sind im Bereich des proximalen Endes 105 an dem Kapillaransatz 102 gelagert.

Zur Lagerung des Klemmelements 302 weist der Kapillaransatz 102 eine Aufnahmeaussparung 107 auf. Die Aufnahmeaussparung 107 ist proximal offen. Die Aufnahmeaussparung 107 erstreckt sich ausgehend von dem proximalen Ende 105 in distaler Richtung in den Kapillaransatz 102. Der Kapillaransatz und/oder die Aufnahmeaussparung 107 weist eine nicht näher bezeichnete proximale Öffnung auf, durch welche das Klemmelement 302 in distaler Richtung in die Aufnahmeaussparung 107 eingesetzt ist. Die Aufnahmeaussparung 107 ist vorliegend koaxial zu dem Lumen 303 und/oder dem Kapillarrohr 101 und/oder dem Katheterschlauch 200 längserstreckt.

Die Aufnahmeaussparung 107 weist eine Innenkonusfläche 108 auf. Die Innenkonusfläche 108 verjüngt sich in distaler Richtung. Die Innenkonusfläche 108 ist wiederum koaxial orientiert. Die Innenkonusfläche 108 wirkt mit einer wenigstens abschnittsweisen komplementären Außenkonusfläche 304 des Klemmelements 302 zusammen. Die Außenkonusfläche 304 verjüngt sich ebenfalls distal und ist in der Klemmkonfiguration an die Innenkonusfläche 108 angepresst. Durch die besagte Anpressbewegung wird das Klemmelement 302 elastisch deformiert das Lumen 303 in radialer Richtung verengt. Diese Verengung führt zu einem (verstärkten) Reibschluss mit dem Katheterschlauch 200, so dass dieser in axialer Richtung reibschlüssig in dem Lumen 303 geklemmt ist.

Das Klemmelement 302 weist ein proximales Ende 305 und ein distales Ende 306 auf (siehe Fig. 3). Die Außenkonusfläche 304 ist zwischen dem proximalen Ende 305 und dem distalen Ende 306 längserstreckt. Bei der gezeigten Ausführungsform ragt das proximale Ende 305 proximal aus der Aufnahmeaussparung 107 heraus. Das Lumen 303 ist durchgängig zwischen dem proximalen Ende 305 und dem distalen Ende 306 längserstreckt und weist eine proximale Einführöffnung 307 und eine distale Austrittsöffnung 308 auf. Die proximale Einführöffnung weist einen ersten Durchmesser D1 auf. Die distale Austrittsöffnung 308 weist einen zweiten Durchmesser D2 auf. Der erste Durchmesser D1 ist größer als der zweite Durchmesser D2. Der vergleichsweise größere erste Durchmesser D1 erleichtert das axiale Einführen des Katheterschlauchs 200 in das Lumen 303. Zudem mündet die proximale Einführöffnung 307 vorliegend in einen distal sich verjüngenden Innenkonus 309 des Lumens 303. Der Innenkonus 309 bildet einen Lumenabschnitt des Lumens 303. Der Innenkonus 309 erlaubt ein weiter vereinfachtes Einführen des Katheterschlauchs 200.

Das Betätigungselement 301 ist bei der gezeigten Ausführungsform schraubbeweglich an dem Kapillaransatz 102 gelagert. Bei einer Bewegung im Uhrzeigersinn wird das Betätigungselement 301 distal auf den Kapillaransatz 102 aufgeschraubt. Das mit dem Betätigungselement 301 wirkverbundene Klemmelement 302 wird infolge der besagten Bewegung des Betätigungselements 301 distal in die Aufnahmeaussparung 107 hineinbewegt. Dies führt zu der zuvor erläuterten Interaktion zwischen der Innenkonusfläche 108 und der Außenkonusfläche 304, so dass das Lumen 303 radial verengt und der Katheterschlauch 200 geklemmt werden.

Zwecks schraubbeweglicher Lagerung weist das Betätigungselement 301 einen Gewindeabschnitt G1 auf. Der Gewindeabschnitt G1 wirkt mit einem komplementären Gewindeabschnitt G2 des Kapillaransatzes 102 zusammen. Bei der gezeigten Ausführungsform ist der Gewindeabschnitt G1 des Betätigungselements 301 ein Innengewinde IG. Der komplementäre Gewindeabschnitt G2 ist dementsprechend ein Außengewinde AG. Das Außengewinde AG ist proximal an dem Kapillaransatz 102 angeordnet. Vorliegend grenzt das Außengewinde AG unmittelbar an das proximale Ende 105 des Kapillaransatzes 102 an. Beide Gewindeabschnitte G1, G2 sind koaxial zu dem Lumen 303 und/oder der gemeinsamen Längsachse X orientiert.

Bei der gezeigten Ausführungsform weist das Betätigungselement 301 einen Kragenabschnitt 310 auf. Der Kragenabschnitt 310 ist koaxial zu dem Lumen 303 orientiert und umgreift das proximale Ende 105 des Kapillaransatzes 102 in Umfangsrichtung. Der Gewindeabschnitt G1, vorliegend im Speziellen das Innengewinde IG, ist an einer nicht näher bezeichneten Innenseite des Kragenabschnitts 310 ausgebildet. Bei der gezeigten Ausführungsform weist der Kragenabschnitt 310 mehrere in Umfangsrichtung zueinander versetzt angeordnete radiale Vorsprünge 311 auf (siehe Fig. 1). Zwecks Drehbetätigung wird der Betätigungsabschnitt 301 zwischen den Fingern einer Hand gegriffen. Die radialen Vorsprünge 311 wirken einem Abrutschen der Finger entgegen.

Bei der gezeigten Ausführungsform ist das Betätigungselement 301 axial formschlüssig und in Umfangsrichtung gleitbeweglich mit dem Klemmelement 302 wirkverbunden.

Bei einer in den Figuren nicht gezeigten Ausführungsform sind das Klemmelement und das Betätigungselement fest miteinander verbunden. Bei einer weiteren Ausführungsform ist eine einstückige Verbindung zwischen Klemm- und Betätigungselement vorgesehen, so dass beide Elemente unterschiedliche Abschnitte ein- und desselben Bauteils bilden.

Zur axial formschlüssigen und in Umfangsrichtung gleitbeweglichen Lagerung des Betätigungselements 301 weist das Klemmelement 302 einen Lagerabschnitt 312 auf. Der Lagerabschnitt 312 ist proximal angeordnet. Der Lagerabschnitt 312 grenzt an das proximale Ende 305 an. Der Lagerabschnitt 312 weist eine Gleitfläche 313 und eine Anlaufschulter 314 auf.

Die Gleitfläche 313 ist koaxial zu dem Lumen 303 und/oder der Längsachse X orientiert. Das Klemmelement 302 durchgreift eine Bohrung 315 des Betätigungselements. Die Bohrung 315 weist eine Innenumfangsfläche 316 auf. Die Innenumfangsfläche 316 ist gleitbeweglich an der Gleitfläche 313 aufgenommen. Die Bohrung 315 ist koaxial zu dem Lumen 303 und/oder der Längsachse X orientiert. Entsprechendes gilt für die Innenumfangsfläche 316. Die Anlaufschulter 314 begrenzt die distale Relativbeweglichkeit des Betätigungselements 301 gegenüber dem Klemmelement 302. Bei einer im Uhrzeigersinn gerichteten Drehbetätigung des Betätigungselements 301 in Richtung der Klemmkonfiguration gelangt das Betätigungselement 301, im Speziellen ein nicht näher bezeichneter Rand der Bohrung 315, formschlüssig zur Anlage an der Anlaufschulter 314. Der Lagerabschnitt 312 weist bei der gezeigten Ausführungsform eine weitere Anlaufschulter 317 auf. Die Anlaufschulter 317 kann auch als proximale Anlaufschulter bezeichnet werden und begrenzt die proximale Relativbeweglichkeit des Betätigungselements 301. Dementsprechend kann die Anlaufschulter 314 auch als distale Anlaufschulter bezeichnet werden. Das Betätigungselement 301 ist im Bereich der Bohrung 315 axial formschlüssig zwischen den beiden Anlaufschultern 314, 317 gehalten.

Bei der gezeigten Ausführungsform ist das Klemmelement 302 aus einem elastomeren Kunststoffmaterial K1 gefertigt. Das Betätigungselement ist aus einem Kunststoffmaterial K2 gefertigt. Das Kunststoffmaterial K2 ist im Vergleich zu dem elastomeren Kunststoffmaterial K1 formstabil.

Um die Fixiervorrichtung 300 ausgehend von der in den Figuren gezeigten Klemmkonfiguration in die Freigabekonfiguration zu überführen, wird das Betätigungselement 301 entgegen dem Uhrzeigersinn betätigt. Hierdurch wird das Betätigungselement 301 in proximaler Richtung relativ zu dem Kapillaransatz 102 schraubbeweglich verlagert. Die Innenumfangsfläche 316 der Bohrung 315 gleitet hierbei in Umfangsrichtung auf der Gleitfläche 313 des Lagerabschnitts 312 des Klemmelements 302 ab. Gleichzeitig drückt das Betätigungselement 301 gegen die proximale Anlaufschulter 317, so dass das Klemmelement 302 gemeinsam mit dem Betätigungselement 301 in proximale Richtung verlagert wird. Infolge der proximalen Verlagerung wird die Wirkverbindung zwischen der Außenkonusfläche des Klemmelements 302 und der Innenkonusfläche 107 aufgehoben. Das Klemmelement 302 wird in radialer Richtung nach außen elastisch entspannt. Dies führt zu einer radialen Aufweitbewegung des Lumens 303. Durch diese Aufweitung wird der Reibschluss zwischen dem Lumen 303 und dem Katheterschlauch 200 aufgehoben oder wenigstens verringert. Der aufgehobene oder wenigstens verringerte Reibschluss gibt die axiale Beweglichkeit des Katheterschlauchs 200 frei, so dass die axiale Länge L situationsgerecht angepasst werden kann.

Nach erfolgter Anpassung der axialen Länge L kann der Katheterschlauch 200 erneut in seiner Beweglichkeit fixiert werden. Hierfür wird das Betätigungselement 301 im Uhrzeigersinn drehbetätigt. Die Innenumfangsfläche 316 gleitet wiederum auf der Gleitfläche 313 in Umfangsrichtung ab. Der nicht näher bezeichnete Rand der Bohrung 315 gelangt zur Anlage an der distalen Anlaufschulter 314 und drückt hierdurch das Klemmelement 302 in die Aufnahmeaussparung 107. Hierdurch wird die Außenkonusfläche 304 an die Innenkonusfläche 108 angepresst. Das Anpressen bewirkt eine elastische Deformation des Klemmelements 302 derart, dass das Lumen 303 in radialer Richtung verengt wird. Durch die Verengung des Lumens 303 wird der Katheterschlauch 200 geklemmt und in seiner relativen axialen Beweglichkeit fixiert.

## Patentansprüche

1. Katheteranordnung (1) zur Verwendung bei einer peripheren Regionalanästhesie, aufweisend
ein Kapillar (100) mit einem Kapillarrohr (101) und einem Kapillaransatz (102), welcher an ein proximales Rohrende (103) des Kapillarrohrs (101) angefügt ist,
einen Katheterschlauch (200), welcher in distaler Richtung durch den Kapillaransatz (102) in das Kapillarrohr (101) eingeschoben ist, wobei ein distales Schlauchende (201) des Katheterschlauchs (200) um eine axiale Länge (L) aus einem distalen Rohrende (104) des Kapillarrohrs (101) hervorsteht,
**dadurch gekennzeichnet, dass** eine Fixiervorrichtung (300) mit einem Betätigungselement (301) und einem Klemmelement (302) vorhanden ist,
wobei das Klemmelement (302) an dem Kapillaransatz (102) gelagert ist,
wobei das Betätigungselement (301) relativ zu dem Kapillaransatz (102) beweglich an dem Kapillaransatz (102) gelagert und kraft- und/oder bewegungsübertragend mit dem Klemmelement (302) wirkverbunden ist,
und wobei das Klemmelement (302) mittels einer Bewegung des Betätigungselements (301) überführbar ist zwischen einer Klemmkonfiguration, in welcher eine axiale Beweglichkeit des Katheterschlauchs (200) unter Einwirkung des Klemmelements (302) fixiert ist, und einer Freigabekonfiguration, in welcher die Einwirkung aufgehoben ist und die axiale Beweglichkeit des Katheterschlauchs (200) freigegeben ist.

2. Katheteranordnung (1) nach Anspruch 1, **dadurch gekennzeichnet, dass** das Klemmelement (302) formnachgiebig ist und ein koaxial zu dem Kapillarrohr (101) ausgerichtetes Lumen (303) aufweist, durch welches der Katheterschlauch (200) längserstreckt ist, wobei das Klemmelement (302) mittels der Bewegung des Betätigungselements (301) derart elastisch deformierbar ist, dass das Lumen (303) in der Klemmkonfiguration unter reibschlüssiger Fixierung des Katheterschlauchs (200) radial verengt ist und der Reibschluss in der Freigabekonfiguration aufgehoben ist.

3. Katheteranordnung (1) nach Anspruch 1 oder 2, **dadurch gekennzeichnet, dass** das Betätigungselement (301) axial schraubbeweglich an dem Kapillaransatz (102) gelagert ist und einen Gewindeabschnitt (G1) aufweist, welcher mit einem komplementären Gewindeabschnitt (G2) des Kapillaransatzes (102) zusammenwirkt.

4. Katheteranordnung (1) nach Anspruch 3, **dadurch gekennzeichnet, dass** der Gewindeabschnitt (G1) als Innengewinde (IG) und der komplementäre Gewindeabschnitt (G2) als Außengewinde (AG) gestaltet ist.

5. Katheteranordnung (1) nach einem der Ansprüche 2 bis 4, **dadurch gekennzeichnet, dass** das Betätigungselement (301) einen Kragenabschnitt (310) aufweist, welcher koaxial zu dem Lumen (303) orientiert ist und ein proximales Ende (105) des Kapillaransatzes (102) in Umfangsrichtung umgreift.

6. Katheteranordnung (1) nach einem der vorhergehenden Ansprüche, **dadurch gekennzeichnet, dass** das Klemmelement (302) in einer proximal offenen Aufnahmeaussparung (107) des Kapillaransatzes (102) aufgenommen ist.

7. Katheteranordnung (1) nach Anspruch 6, **dadurch gekennzeichnet, dass** das Klemmelement (302) eine koaxial zu dem Lumen (303) orientierte und distal sich verjüngende Außenkonusfläche (304) aufweist, wobei in der Klemmkonfiguration die Außenkonusfläche (304) gegen eine Innenkonusfläche (108) der Aufnahmeaussparung (107) gepresst ist.

8. Katheteranordnung (1) nach einem der Ansprüche 2 bis 7, **dadurch gekennzeichnet, dass** das Lumen (303) zwischen einer proximalen Einführöffnung (307) mit einem ersten Durchmesser (D1) und einer distalen Austrittsöffnung (308) mit einem zweiten Durchmesser (D2) längserstreckt ist, wobei der erste Durchmesser (D1) größer ist als der zweite Durchmesser (D2).

9. Katheteranordnung (1) nach Anspruch 8, **dadurch gekennzeichnet, dass** die proximale Einführöffnung (307) in einen distal sich verjüngenden Innenkonus (309) des Lumens (303) mündet.

10. Katheteranordnung (1) nach einem der vorhergehenden Ansprüche, **dadurch gekennzeichnet, dass** das Betätigungselement (301) axial formschlüssig und in Umfangsrichtung gleitbeweglich an dem Klemmelement (302) gelagert ist.

11. Katheteranordnung (1) nach einem der Ansprüche 2 bis 10, **dadurch gekennzeichnet, dass** das Klemmelement (302) einen proximal angeordneten Lagerabschnitt (312) mit einer koaxial zu dem Lumen (303) orientierten Gleitfläche (313) und einer die Gleitfläche (313) distal begrenzenden Anlaufschulter (314) aufweist, wobei der Lagerabschnitt (312) eine koaxial zu dem Lumen (303) orientierte Bohrung (315) des Betätigungselements (301) axial durchgreift, und wobei eine Innenumfangsfläche (316) der Bohrung (315) gleitbeweglich auf der Gleitfläche (313) abgestützt ist.

12. Katheteranordnung (1) nach einem der vorhergehenden Ansprüche, **dadurch gekennzeichnet, dass** das Klemmelement (302) aus einem elastomeren Kunststoffmaterial (K1) gefertigt ist und/oder dass das Betätigungselement (301) aus einem im Vergleich zu dem Klemmelement (302) formstabilen Kunststoffmaterial (K2) gefertigt ist.
